# FASCICULE DE BREVET EUROPEEN

(11) **EP 1 002 524 B1**
(45) Date de publication et mention de la délivrance du brevet: **19.02.2003**
(21) Numéro de dépôt: 98402877.9
(22) Date de dépôt: 20.11.1998
(51) Int. Cl.: A61K 7/48

(54) **Composition ayant une action anti-vieillissement de la peau**
Hautalterungsschutzmittel
Skin antiageing composition

(43) Date de publication de la demande: 24.05.2000
(73) Titulaire: Marie Pratt Sarl, 91170 Viry Chatillon (FR)
(72) Inventeur: Lerondeau, Claire, 91170 Viry Chatillon (FR); Monclus, Dominique, 92170 Vanves (FR)
(74) Mandataire: Hammond, William

(56) Documents cités:
- EP-A- 0 467 218
- WO-A-90/01323
- WO-A-91/11169
- DE-A- 19 651 428
- FR-A- 2 642 966
- DATABASE WPI Week 9840 Derwent Publications Ltd., London, GB; AN 98-462767 XP002100486 & JP 10 194959 A (KANEBO LTD) , 28 juillet 1998
- DATABASE WPI Week 9544 Derwent Publications Ltd., London, GB; AN 95-340173 XP002100487 & JP 07 233044 A (DOWA MINING CO. LTD.) , 5 septembre 1995

## Description

La présente invention est relative à une composition ayant une action anti-vieillissement de la peau.

Au fil des jours, la peau vieillit ce qui se manifeste, par exemple, par l'apparition et le développement de rides notamment sur le visage, et/ou par un affaissement des joues et/ou des paupières. On note concomitamment une désorganisation de la partie profonde de la peau, un ralentissement du renouvellement cellulaire ainsi qu'une production de collagène et élastine, de mauvaises qualités.

Outre la cause hormonale du vieillissement, il existe également des causes dites externes telles que :
- le soleil, le tabac, le manque de sommeil,
- des régimes alimentaires déficients en vitamines : ainsi à partir de cinquante ans, il est préférable d'éviter les graisses animales qui augmentent le taux de cholestérol dans le sang et accroissent les risques de maladies cardio-vasculaires.

Depuis de nombreuses années, on connaît des produits cosmétiques pour combattre le vieillissement de la peau. Ainsi, le document FR-A-2.664.162 est relatif à une composition cosmétique utilisable notamment pour le maquillage du visage et susceptible de protéger la peau contre les agressions de l'air atmosphérique. Cette composition contient une association d'huiles végétales de barbassu et de rosier sauvage, de perfluoropolyéther, de δ-orizanol, de sels de l'acide éthylènediamine tétracétique, de céthyl phosphate de potassium, d'acide 18β glyccyrhétinique et d'extrait glycolique de miel. Selon ce document de l'art antérieur, les huiles végétales de barbassu et de rosier sauvage sont stables à l'oxydation et atténuent une attaque oxydante du milieu ambiant. Mais, un tel produit ne constitue pas une réponse satisfaisante pour lutter contre le vieillissement de la peau.

Aussi, un des buts de la présente invention est-il de fournir une composition anti-vieillissement de la peau, qui permet en particulier, d'enrayer l'apparition de rides et d'améliorer l'aspect de la peau.

Un autre but de l'invention est de fournir une telle composition utilisable en application topique et/ou ingérable.

Ces buts ainsi que d'autres qui apparaîtront par la suite, sont atteints par une composition ayant notamment une action anti-vieillissement de la peau comprenant un complexe d'huiles végétales riches en acides gras essentiels, laquelle composition est caractérisée, selon la présente invention, par le fait que ce complexe contient de l'huile de rosier muscat, d'huile de camélia et d'huile de tournesol et qu'elle renferme également de la vitamine E.

Avantageusement, le complexe d'huiles végétales riches en acides gras essentiels contient aussi de l'huile de fleur de la passion.

De préférence, cette composition contient de 0,1 à 30 % en poids du complexe et de 0,1 à 10 % du poids en vitamine E.

Avantageusement, le complexe renferme au plus 10 % en poids d'huile de rosier muscat, au plus 10 % en poids d'huile de camélia et au plus 10 % en poids d'huile de tournesol. La quantité d'huile de fleur de la passion lorsque celle-ci est présente dans le complexe, est comprise entre 0 et 10 % en poids.

Les acides gras poly-insaturés sont des constituants naturels des graisses animales et végétales. Dans l'organisme, ils servent, soit de réserve énergétique, soit de composants des membranes cellulaires. Ils sont également, les précurseurs de molécules actives comme certaines prostaglandines.

Ces acides gras sont dits poly-insaturés car ils contiennent plusieurs doubles liaisons dans leur formule chimique. Ils sont classés en fonction de la position de leur première double liaison. Ainsi, les acides gras poly-insaturés n-3 encore dénommés Oméga 3 ou ω3, ont leur première double liaison placée à trois atomes de carbone de la terminaison méthyle (CH₃) de la molécule. Les acides gras poly-insaturés n-6 (Oméga 6 ou ω6) ont leur première double liaison à six atomes de carbone de cette même terminaison.

Certains acides gras poly-insaturés sont dits « essentiels » car l'organisme humain ne sait pas les synthétiser : ils doivent donc être apportés par l'alimentation. Ce sont les acides linoléique (Oméga 6) et alpha-linolénique (Oméga 3). Dans l'organisme, ils peuvent être mis en réserve, fournir de l'énergie ou bien encore être transformés. Dans ce dernier cas, ces deux acides gras essentiels sont convertis en dérivés actifs, sous l'action d'enzymes communes.

Les principaux dérivés actifs de l'acide linoléique sont les acides gammalinolénique (GLA) et arachinonique. Ceux de l'acide alpha-linolénique sont l'acide eicosapentaénoïque (EPA) et l'acide docosahexaénoïque (DHA).

Chez l'homme, les réactions aboutissant à ces dérivés actifs, par le biais des enzymes communes sont lentes et limitées. En particulier, dans la période périnatale, ces enzymes semblent avoir une activité insuffisante pour fournir la quantité d'acides gras dits poly-insaturés nécessaire à l'élaboration des membranes, y compris cérébrales.

Lors du vieillissement et de certaines pathologies (maladies endocriniennes, alcoolisme...), ces enzymes sont parfois altérées. Il est donc souhaitable que l'alimentation fournisse, non seulement les acides gras essentiels mais aussi leurs dérivés (GLA, EPA, DHA...), en quantité adaptée.

Les acides gras dits poly-insaturés sont surtout présents dans les végétaux. Les huiles de soja, de maïs, de noix, de tournesol, de bourrache, de rosier muscat, etc. en contiennent beaucoup. L'huile de rosier muscat utilisée dans la présente invention contient en poids environ 41 % d'acide linoléique et 39 % de linolénique.

Les acides gras essentiels (Oméga 3 et Oméga 6) ont non seulement un rôle structural au niveau des phospholipides membranaires dont ils conditionnent la fluidité, mais aussi un rôle biologique, puisque certains d'entre eux sont les précurseurs d'eicosanoïdes (protaglandines et leucotriènes) dont le rôle dans les phénomènes inflammatoires et immunitaires est fondamental.

Dans le cas de déficience en acides gras essentiels chez l'homme, les manifestations cliniques peuvent apparaître plus ou moins rapidement par rapport aux signes biologiques : il s'agit principalement de signes cutanés avec perte de souplesse et d'élasticité de la peau, xérodermie et desquamation exagérée. Mais, une atrophie peut être observée ainsi que des lésions érythémato-squameuses. Enfin, il existe parfois un retard de cicatrisation, de purpura, par fragilité capillaire.

L'huile naturelle de rosier muscat est obtenue à partir du cynorrhodon, rose sauvage dont le nom scientifique est Rosa aff. Rubiginosa qui pousse abondamment dans les Andes et au Chili.

Cette huile a une teneur élevée en acides gras essentiels, 80 %, en poids comprenant :
44 à 50 % d'acide linoléique (C18:2)
30 à 35 % d'acide linolénique (C18:3)
16 % d'acide oléique (C18:1)

Ce type d'acides gras est essentiel pour la synthèse de la prostaglandine, des membranes cellulaires, les mécanismes de défense, de croissance et dans les processus physiologiques et biochimiques impliqués dans la régénération cellulaire.

Les caractéristiques techniques et propriétés physiques de l'huile de rosier muscat sont :
Viscosité à 20°C : 42,3
Indice d'iode : 181,2
Indice de saponification : 190,0
Indice d'acidité : 0,82
Densité à 15°C : 0,9327
Équivalent de saponification : 893,37
Indice d'ester : 189,18
% de gras neutre : 99,57
% de gras acide : 0,43
% de résidu insaponifiable : 0,76
Indice de réfraction à 20°C : 1,4793
Viscosité à 20°C (cp) : 42,30
Viscosité cinématique à 20°C (cst) : 45,83
Point de fige : -17,8°C (à rapprocher de : huile d'olive : - 12,2°C ;
   huile de lin : - 17,8°C)

C'est pourquoi, cette huile naturelle donne d'excellents résultats en cosmétique dans le maintien et la fraîcheur de la peau, la prévention du vieillissement, l'atténuation des cicatrices et des taches, les peaux sèches et très sèches, les vergetures.

Le tournesol, fleur de soleil, Hélianthus, est une grande plante dont le capitule floral, graine d'or, renferme des grains riches en huile.

Cette plante, originaire d'Amérique est couramment cultivée en Europe.

L'huile de tournesol est naturellement riche en acides gras insaturés (87 %) et contient également 1 % d'insaponifiables.

Les caractéristiques techniques de l'huile de tournesol sont :
Composition moyenne de l'insaponifiable en mg/g d'huile
   Alcools terpéniques : 100-150
   Stérols : 300-400
   dont:
      campestérol 11 %
      β-sitostérol 61 %
      stigmastérol 8 %
      δ-7 stigmastérol 20 %
   Aspect : liquide
   Couleur : jaunâtre
   Densité à 20°C : 0,835
   Solubilité dans l'eau : insoluble
   Solubilité dans l'huile : soluble
   Point éclair : > 113°C

L'huile végétale de camélia est extraite de graines de fleur de camélia, ce bel arbre qui donne les premières fleurs de l'année, d'où son nom qui annonce le Printemps.

Elle contient principalement de l'acide oléique. Elle complète remarquablement une association d'autres huiles végétales, riches en acide gras essentiels.

Les caractéristiques techniques de l'huile végétale de Camélia sont :
Densité à 20°C : 0,915 + 0,010
Indice de réfraction à 20°C : 1,4650 - 1,4750
Indice d'acide : < 0,50
Indice d'iode : 78-88
Indice de peroxyde : < 2

Elle est, par conséquent, particulièrement intéressante pour ses effets nutritifs en application cutanée.

L'huile naturelle de fleur de la passion est obtenue à partir d'une espèce tropicale de fleur de la passion, originaire du Brésil, d'Australie et de certaines régions d'Afrique, dont le nom scientifique est passiflora incarnata ou passiflora edulis.

L'huile est extraite de semences, par pression ; elle est riche en acides gras essentiels notamment :
70 % d'acide linoléique (C18:2)
16 % d'acide oléique (C18:1)
10 % d'acide palmitique (C16:0)
3 % d'acide stéarique (C18:0)

Utilisée sur la peau, cette huile végétale et naturelle y dépose un film soyeux, lui donnant ainsi souplesse et douceur.

Les Caractéristiques techniques de l'huile naturelle de fleur de la passion sont :
Indice de réfraction à 20°C : 1,468-1,469
Indice d'acide : < 0,2
Indice d'iode : 137-147
Saponification : 188-194
Insaponifiables : 0,8-2,0 %
Acide palmitique : 9
Acide oléique : 13
Acide linoléique : 76

La vitamine E ou α-tocophérol est une vitamine liposoluble essentielle qui fût découverte en 1922 par Evans et Bishop.

L'α-tocophérol, en particulier le DL α-tocophérol présent dans la nature est celui qui présente l'activité biologique la plus élevée.

A titre d'exemples non limitatifs, diverses formations du complexe selon l'invention et du produit le contenant sont indiqués ci-après (les compositions sont exprimées en poids %).

### Exemple n° 1 : Crème nourrissante pour le visage

- huile de rosier muscat 10 %
- huile de fleur de la passion 0,5 %
- huile de tournesol 0,5 %
- huile de camélia 1 %
- DL α-tocophérol acétate 0,5 %
- phase grasse 31,78 %
- conservateur 0,3 %
- eau purifiée QSP

### Exemple n° 2 : Crème peaux jeunes matifiante

- huile de rosier muscat 1 %
- huile de fleur de la passion 0,5 %
- huile de tournesol 0,5 %
- huile de camélia 0,5 %
- DL α-tocophérol acétate 0,5 %
- phase grasse 24,74 %
- conservateur 0,3 %
- eau purifiée QSP

### Exemple n° 3 : Gel hydratant

- huile de rosier muscat 0,5 %
- huile de fleur de la passion 0,5 %
- huile de tournesol 0,5 %
- huile de camélia 0,5 %
- DL α-tocophérol acétate 0,5 %
- phase grasse 57,1 %
- conservateur 0,3 %
- eau purifiée QSP

### Exemple n° 4 : Hydrogel démaquillant pour maquillage

PHASE GEL QSP PHASE EMULSION
- huile de rosier muscat 0,5 %
- huile de fleur de la passion 0,5 %
- huile de tournesol 0,5%
- huile de camélia 0,5 %
- DL α-tocophérol acétate 0,5 %
- phase grasse 15,7 %
- eau purifiée QSP

### Exemple n° 5 : Huile triphasée démaquillante

- huile de rosier muscat 1 %
- huile de fleur de la passion 0,1 %
- huile de tournesol 0,1 %
- huile de camélia 0,1 %
- DL α-tocophérol acétate 0,5 %
- phase grasse 50,5 %
- eau purifiée QSP

### Exemple n° 6 : Masque

- huile de rosier muscat 10 %
- huile de fleur de la passion 5 %
- huile de tournesol 4 %
- huile de camélia 3 %
- DL α-tocophérol acétate 5%
- phase grasse 42,6 %
- conservateur 0,3 %
- eau purifiée QSP

### Exemple n° 7 : Voile corporel

- huile de rosier muscat 5 %
- huile de fleur de la passion 2 %
- huile de tournesol 1 %
- huile de camélia 1 %
- DL α-tocophérol acétate 1 %
- phase grasse 23,68 %
- conservateur 0,3 %
- eau purifiée QSP

### Exemple n° 8 : Buste fermeté

- huile de rosier muscat 10 %
- huile de fleur de la passion 5 %
- huile de tournesol 5 %
- huile de camélia 5 %
- DL α-tocophérol acétate 10 %
- phase grasse 13,27 %
- eau purifiée QSP

### Exemple n° 9 : Gel solaire semifluide

- huile de rosier muscat 4 %
- huile de fleur de la passion 2 %
- huile de tournesol 2 %
- huile de camélia 2 %
- DL α-tocophérol acétate 1 %
- phase grasse QSP

### Exemple n° 10 : Ecran total

- huile de rosier muscat 5 %
- huile de fleur de la passion 1 %
- huile de tournesol 1 %
- huile de camélia 1 %
- DL α-tocophérol acétate 0,5 %
- phase grasse 59,74 %
- conservateur 0,25 %
- eau purifiée QSP

### Exemple n° 11 : Capsules nutrition fermeté

- huile de camélia 5 mg (1,187 %)
- huile de tournesol 255 mg (60,4 %)
- huile de fleur de la passion 100 mg (23,53 %)
- huile de rosier muscat 60 mg (14,12 %)
- DL α-tocophérol acétate 5 mg (1,18 %)

Par ailleurs, des essais ont été réalisés pour mettre en évidence la synergie entre les huiles végétales contenues dans les acides gras poly-insaturés et la vitamine E.

Pour cela, on a réalisé les trois compositions suivantes :
- Produit A :: excipient contenant 5 % de palmitate de vitamine E.
- Produit B :: excipient contenant 5 % d'un mélange d'huiles végétales de rosier muscat, camélia, tournesol.
- Produit C :: excipient contenant 5 % de palmitate de vitamine E, 5 % du mélange d'huiles végétales de rosier muscat, camélia, tournesol.

On a mesuré l'hydratation de la peau de patients qui ont ingéré ces compositions et/ou appliqué sur la peau ces compositions. Les patients ont été groupés en trois groupes, chaque groupe testant l'un des produits.

La courbe d'hydratation a été obtenue en calculant la moyenne des valeurs d'hydratation correspondant aux patients d'un même lot, pour chaque produit testé, et ce pour toutes les heures jusqu'à 6 heures. Cette courbe constitue la figure unique en annexe.

Pour le produit A : l'augmentation d'hydratation atteint une valeur de 15, à la première heure.

Ensuite, l'hydratation redescend très vite, et se stabilise vers une valeur moyenne de 5, jusqu'à la sixième heure. Le produit A n'hydrate pas la peau de façon significative.

Pour le produit B : l'augmentation d'hydratation atteint une valeur 15, dès la première heure, puis redescend plus progressivement vers une valeur moyenne de 12, jusqu'à la sixième heure. Le produit B hydrate la peau significativement, de façon modérée.

Pour le produit C : l'hydratation atteint une valeur de 15, dès la première heure. Cette valeur augmente dès la deuxième heure et continue d'augmenter progressivement jusqu'à une valeur de 20. A la sixième heure, bien que le taux d'hydratation commence à descendre, les valeurs sont toujours voisines de 20.

Par conséquent, l'essai C apporte une hydratation importante et constante pendant six heures.

Cette étude montre que le produit selon la présente invention présente une activité bien supérieure à celle du produit ne renfermant que l'un ou l'autre des constituants.

Il doit être précisé que dans le cas d'une composition ingérable, celle-ci contient de 0,1 à 3 % en poids d'huile de camélia, de 50 à 70 % en poids d'huile de tournesol, de 12 à 17 % en poids d'huile de rosier muscat, de 0 à 26 % en poids d'huile de fleur de la passion et 1 à 5 % en poids de vitamine E.

## Revendications

1. Composition ayant une action anti-vieillissement de la peau comprenant un complexe d'huiles végétales riches en acides gras essentiels, **caractérisée par le fait que** ledit complexe contient de l'huile de rosier muscat, d'huile de camélia et d'huile de tournesol et qu'elle renferme également de la vitamine E.

2. Composition selon la revendication 1, **caractérisée par le fait que** le complexe d'huiles végétales riches en acides gras essentiels contient aussi de l'huile de fleur de la passion.

3. Composition, selon les revendications 1 et 2, **caractérisée par le fait qu'**elle contient de 0,1 à 30 % en poids du complexe et 0,1 à 10 % en poids de vitamine E pour une préparation en application topique.

4. Composition selon la revendication 1, **caractérisée par le fait que** le complexe renferme au plus 10 % en poids d'huile de rosier muscat, au plus 10 % en poids d'huile de camélia et au plus 10 % en poids d'huile de tournesol pour une préparation en application topique.

5. Composition, selon la revendication 2, **caractérisée par le fait que** le complexe contient également de 0 à 10 % en poids d'huile de fleur de la passion pour une préparation en application topique.

6. Composition, selon l'une quelconque des revendications 1 ou 2, **caractérisée par le fait qu'**elle contient, pour une application ingérable, de 0,1 à 3 % en poids d'huile de camélia, de 50 à 70 % en poids d'huile de tournesol, de 12 à 17 % en poids d'huile de rosier muscat, de 0 à 26 % en poids d'huile de fleur de la passion et 1 à 5 % en poids de vitamine E.

## Claims

1. Composition having an anti-ageing action on skin, comprising a complex of vegetable oils rich in essential fat acids, **characterized by** the fact the aforementioned complex contains some rosehips, camellia and sunflower oils and that it also holds some vitamin E.

2. Composition according to claim 1, **characterized by** the fact that the complex of vegetable oils rich in essential fat acids also contains some passionflower oil.

3. Composition according to claims 1 and 2, **characterized by** the fact that it contains from 0,1 to 30 % in weight of the complex and 0,1 to 10 % in weight of vitamin E for a preparation in topical application.

4. Composition according to claim 1, **characterized by** the fact that the complex holds at the most 10 % in weight of rosehips oil, at the most 10 % in weight of camellia oil and at the most 10 % in weight of sunflower oil for a preparation in topical application.

5. Composition according to claim 2, **characterized by** the fact that the complex also contains from 0 to 10 % in weight of passionflower for a preparation in topical application.

6. Composition according to anyone of claim 1 or 2, **characterized by** the fact that it contains for an ingestable application from 0,1 to 3 % in weight of camellia oil, from 50 to 70 % in weight sunflower oil, from 12 to 17 % in weight of rosehips oil, from 0 to 26 % in weight of passionflower oil and 1 to 5 % in weight of vitamin E.

## Patentansprüche

1. Zusammensetzung, die insbesondere über eine Anti-Ageingwirkung auf die Haut verfügt und einen Pflanzenölkomplex mit vielen essentiellen Fetten enthält, und die **dadurch gekennzeichnet ist, dass** dieser Komplex Muskatrosenöl, Kamelienöl und Sonnenblumenöl enthält und dass sie ebenfalls Vitamin E beinhaltet.

2. Zusammensetzung gemäß Anspruch 1, **dadurch gekennzeichnet, dass** der Pflanzenölkomplex, der reich an essentiellen Fettsäuren ist, ebenfalls Passionsblumenöl enthält.

3. Zusammensetzung gemäß den Ansprüchen 1 und 2, **dadurch gekennzeichnet, dass** sie gewichtsmäßig 0,1 bis 30% des Komplexes und gewichtsmäßig 0,1 bis 10% Vitamin E für eine Präparation zur topischen Anwendung enthält.

4. Zusammensetzung gemäß Anspruch 1, **dadurch gekennzeichnet, dass** der Komplex gewichtsmäßig höchstens 10% Muskatrosenöl, gewichtsmäßig höchstens 10% Kamelienöl und gewichtsmäßig höchstens 10% Sonnenblumenöl für eine Präparation zur topischen Anwendung enthält.

5. Zusammensetzung gemäß Anspruch 2, **dadurch gekennzeichnet, dass** der Komplex ebenfalls gewichtsmäßig 0 bis 10% Passionsblumenöl für eine Präparation zur topischen Anwendung enthält

6. Zusammensetzung gemäß einem der Ansprüche 1 oder 2, **dadurch gekennzeichnet, dass** sie für eine ingerierbare Anwendung gewichtsmäßig 0,1 bis 3% Kamelienöl, gewichtsmäßig 50 bis 70% Sonnenblumenöl, gewichtsmäßig 12 bis 17% Muskatrosenöl, gewichtsmäßig 0 bis 26% Passionsblumenöl und gewichtsmäßig 1 bis 5% Vitamin E enthält.
